# EUROPÄISCHE PATENTSCHRIFT

(11) **EP 1 398 387 B1**
(45) Veröffentlichungstag und Bekanntmachung des Hinweises auf die Patenterteilung: **18.04.2007**
(21) Anmeldenummer: 03019295.9
(22) Anmeldetag: 26.08.2003
(51) Int. Cl.: C12Q 1/56, G01N 33/86

(54) **Verbesserung der Spezifität bei der Bestimmung von Antithrombin III**
Improvement of specificity in antithrombin III determination methods
Amélioration de spécifité dans des méthodes de détermination de l'antithrombin III

(30) Priorität: 29.08.2002 DE 10239821
(43) Veröffentlichungstag der Anmeldung: 17.03.2004
(73) Patentinhaber: F. HOFFMANN-LA ROCHE AG, 4070 Basel (CH); Roche Diagnostics GmbH, 68305 Mannheim (DE)
(72) Erfinder: Adema, Enno, 69117 Heidelberg (DE)
(74) Vertreter: Weiss, Wolfgang

(56) Entgegenhaltungen:
- EP-A- 0 391 433
- EP-A- 0 657 547
- EP-A- 0 927 767
- US-A- 5 320 945
- US-A- 5 891 647
- PATENT ABSTRACTS OF JAPAN vol. 018, no. 356 (P-1765), 5. Juli 1994 (1994-07-05) & JP 06 094713 A (MASASHI FUNAYAMA), 8. April 1994 (1994-04-08)
- TOPPER M J: "Enzyme-linked immunosorbent assay for thrombin-antithrombin III complexes in horses" AJVR, Bd. 57, Nr. 4, April 1996 (1996-04), Seite 427-431 XP009021666
- RUZZENETE O ET AL: "USE OF PURIFIED DERMATAN SULFATE FOR HEPARIN COFACTOR II HC II ASSAY" THROMBOSIS RESEARCH, Bd. 65, Nr. 2, 1992, Seiten 281-287, XP009021769 ISSN: 0049-3848
- PELZER H ET AL: "DETERMINATION OF HUMAN THROMBIN-ANTITHROMBIN III COMPLEX IN PLASMA WITH AN ELISA" THROMBOSIS AND HAEMOSTASIS, Bd. 59, Nr. 1, 1988, Seiten 101-106, XP009021770 ISSN: 0340-6245
- DEMERS C ET AL: "An antithrombin III assay based on factor Xa inhibition provides a more reliable test to identify congenital antithrombin III deficiency than an assay based on thrombin inhibition" THROMBOSIS AND HAEMOSTASIS, STUTTGART, DE, Bd. 69, Nr. 3, 1993, Seiten 231-235, XP002101236 ISSN: 0340-6245

## Beschreibung

Die Erfindung betrifft ein Verfahren zur Bestimmung von Antithrombin III (AT) in Körperflüssigkeiten durch Zugabe eines AT-Bindepartners zur Probe und Bestimmung des freien AT-Bindepartners sowie ein dafür geeignetes Reagenz.

AT ist ein Faktor des Blutgerinnungssystems, der der Regulation dient. Die Blutgerinnung wird in Gang gesetzt durch das kaskadenartige Zusammenwirken verschiedener Proteasen. Der letzte der hintereinander geschalteten Aktivierungsschritte setzt Thrombin frei, das wiederum durch Spaltung von Fibrinogen Fibrinmonomere erzeugt, die sich zusammenlagern und einen Thrombus bilden. Wichtigster Regulator ist AT, das mit Thrombin und auch mit anderen an der Blutgerinnung beteiligten Proteasen einen Komplex bilden kann, der das aktive Zentrum blockiert. Der AT-Gehalt im Blut liegt bei gesunden Menschen in einem relativ engen Bereich. Verminderte AT-Gehalte können durch eine Verbrauchskoagulopathie, eine schwere Lebererkrankung oder erblich bedingt sein. Ein verminderter AT-Gehalt wird heute allgemein als Thromboserisiko gewertet. Deshalb ist in manchen Fällen der AT-Gehalt auch bei einer akuten Thrombose reduziert. Der AT-Gehalt ist daher ein wertvoller Parameter in der klinischen Diagnostik.

Es sind bereits verschiedene Verfahren zum Nachweis von AT bekannt, bei denen einer Probe ein AT-Bindepartner unter Bedingungen zugesetzt wird, bei denen eine Interaktion des AT-Bindepartners mit in der Probe vorhandenem AT erfolgen kann, und anschließend eine Bestimmung des Anteils von freiem AT-Partner erfolgt. Derartige Bestimmungen können z.B. auf Basis immunologischer Methoden oder unter Verwendung chromogener Substrate erfolgen. Im letzteren Fall wird der Probe z.B. Thrombin oder aktivierter Faktor X zugesetzt, das mit in der Probe vorhandenem AT interagiert. Überschüssiges Thrombin wird dann durch Inkubation mit einem chromogenen Substrat, das durch Einwirkung von Thrombin eine gefärbte Substanz bildet, und Auswertung der Farbbildung nachgewiesen, wobei der AT-Gehalt in indirektem Verhältnis zu der Farbbildung steht. Verfahren zur Bestimmung von AT sind beispielsweise beschrieben in Bergmeyer, "Methods of Enzymatic Analysis", 3d edition, Verlag Chemie, Vol. 5, S. 441-448; I. Witt, ed., "Neue Methoden der Gerinnungsanalyse mit chromogenen Substraten", Stormorken, Neue Methoden der Gerinnungsanalyse, Seite 119-121; Odegard et al., Haemostasis 7: 202-209 (1978); Fareed et al., Chromogenic Peptide Substrates (eds. M.F. Scully and V.V. Kakkar) Churchill Livingstone (1979) 183-191 und Abildgaard et al., Thromb. Res. 11, 549-553 (1977).

Ein Nachteil bekannter Verfahren zum Nachweis von AT durch Zugabe von Thrombin besteht darin, dass in Gegenwart von Störfaktoren, z.B. Arzneimitteln, wie etwa Hirudin, die selbst mit Thrombin interagieren können, ein falscher hoher AT-Wert erhalten wird. Dieser Nachteil kann durch Verwendung von aktiviertem Faktor Xa anstelle von Thrombin vermieden werden. Es befinden sich derzeit jedoch mehrere Faktor Xa-Inhibitoren in der Entwicklung als Therapeutikum (Ostrem et al., Biochemistry 37 (1998), 1053-1059; US-Patent 5,783,421; US-Patent 5,721,214; WO 96/40679; US-Patent 5,693,641; WO 97/46523; JP-96-191434 etc.). Wenn diese Mittel auf den Markt kommen, treten bei einem auf Faktor Xa basierenden Nachweisverfahren die gleichen Probleme wie bei dem auf Thrombin basierenden Test auf.

Es war daher Aufgabe der Erfindung, bekannte Nachweismethoden zu verbessern und ein Verfahren zur Verfügung zu stellen, das auch bei Anwesenheit von Störfaktoren in der zu testenden Probe zu einem zuverlässigen Messergebnis führt.

Diese Aufgabe wird gelöst durch ein Verfahren zum Nachweis von Antithrombin III (AT) in einer Probe, die eventuell einen Störfaktor enthält, umfassend die Schritte:
(a) Inkontaktbringen der Probe mit einem ersten Reagenz R1 enthaltend einen AT-Bindepartner unter Bedingungen, bei denen der AT-Bindepartner im Wesentlichen nicht mit AT interagiert, aber mit dem Störfaktor interagiert,
(b) Zugeben eines zweiten Reagenz R2 zu einer ersten Bestimmung des freien Anteils des AT-Bindepartners,
(c) Zugeben eines dritten Reagenz R3 zur Änderung der Bedingungen, so dass der AT-Bindepartner mit AT interagiert, und zweite Bestimmung des freien Anteils des AT-Bindepartners und
(d) Ermitteln des AT-Gehalts in der Probe aus der Differenz der ersten und zweiten Bestimmung des freien Anteils des AT-Bindepartners.

Das erfindungsgemäße Verfahren umfasst den Nachweis von AT in einer Probe, insbesondere in einer Körperflüssigkeit, wie etwa Blut oder Plasma, basierend auf der Bestimmung der Interaktion eines AT-Bindepartners mit in der Probe vorhandenem AT, wobei man eine erste Bestimmung des AT-Bindepartners ohne AT-Interaktion und danach eine zweite Bestimmung des AT-Bindepartners mit AT-Interaktion durchführt und den AT-Gehalt der Probe aus der Differenz der ersten und der zweiten Bestimmung ermittelt.

Das erfindungsgemäße Verfahren basiert darauf, dass zwei Bestimmungen des freien Anteils von AT-Bindepartner in der Probe bei unterschiedlichen Bedingungen durchgeführt werden. Eine erste Bestimmung des AT-Bindepartners erfolgt ohne AT-Interaktion, d.h. unter Bedingungen, bei denen vorhandenes AT im Wesentlichen nicht, d.h. nicht oder nur in einem die Bestimmung nicht wesentlich beeinträchtigendem Ausmaß, mit dem AT-Bindepartner interagiert, weil AT beispielsweise nicht in aktiver Form vorliegt. Anschließend erfolgt eine Änderung der Bedingungen, so dass in der Probe vorhandenes AT mit dem AT-Bindepartner interagieren kann, indem, beispielsweise durch Zugabe eines geeigneten Reagenz, Bedingungen eingestellt werden, unter denen die Interaktion, z.B. Komplexbildung zwischen AT und AT-Bindepartner, beschleunigt wird. Die nachfolgende zweite Bestimmung des restlichen freien (und aktiven) AT-Bindepartners erlaubt einen Rückschluss auf den AT-Gehalt der Probe.

Die Bestimmung des freien AT-Bindepartners kann grundsätzlich nach jeder beliebigen Methode durchgeführt werden. Bevorzugt sind chromogene Aktivitätsbestimmungen, bei denen beispielsweise eine proteolytische Aktivität von AT-Bindepartnern, wie Thrombin oder Faktor Xa, erfolgt, oder eine immunologische Bestimmung, bei der beispielsweise Antikörper eingesetzt werden, die spezifisch gegen einen nicht (mit AT) komplexierten AT-Bindepartner gerichtet sind, und die anschließende Komplexbildung zwischen AT und AT-Bindepartnern nicht stören.

Eine besonders bevorzugte Ausführungsform des erfindungsgemäßen Verfahrens umfasst eine Bestimmung der proteolytischen Aktivität eines AT-Bindepartners. Dabei wird die nach Reaktion mit einem Störfaktor verbleibende proteolytische Aktivität des AT-Bindepartners unter Bedingungen bestimmt, bei denen in der Probe vorhandenes AT nicht oder nur geringfügig mit dem AT-Bindepartner reagieren kann. Anschließend erfolgt eine Beschleunigung der Komplexbildung zwischen AT und AT-Bindepartner, z.B. durch Aktivierung des AT. Dabei bilden sich aus dem aktivierten AT und dem Bindepartner Komplexe. Ein derartig komplexierter Bindepartner hat im Wesentlichen keine proteolytische Aktivität mehr. Anschließend wird erneut die Aktivität des Bindepartners bestimmt. Die Differenz zwischen der ersten und der zweiten Aktivität entspricht der Menge an AT in der Probe.

Der AT-Bindepartner ist eine nachweisbare Substanz, vorzugsweise eine Substanz mit Protease-Aktivität, die einen Komplex mit AT bilden kann und dadurch vorzugsweise inhibiert wird. Beispiele für geeignete AT-Bindepartner sind Thrombin und Faktor Xa. Besonders bevorzugt verwendet man Thrombin.

Der Nachweis des AT-Bindepartners erfolgt vorzugsweise durch ein chromogenes Substrat, das unter Einwirkung des AT-Bindepartners eine Farbe bildet und Messung der entstehenden Farbe. Beispiele für bevorzugte Substrate sind peptidische Substrate, beispielsweise das Thrombinsubstrat Tos-Gly-Pro-Arg-p-Nitroanilin (Chromozym ®TH), das unter Einwirkung von Thrombin zu Tos-Gly-Pro-Arg-OH und p-Nitroanilin umgesetzt wird. Selbstverständlich sind jedoch auch andere Substrate, die von dem entsprechenden AT-Bindepartner akzeptiert werden, geeignet.

Im Gegensatz zu Verfahren des Standes der Technik erfolgt im erfindungsgemäßen Verfahren eine erste Bestimmung der Aktivität des AT-Bindepartners unter Bedingungen, bei denen in der Probe vorhandenes AT nicht oder nur geringfügig den Bindepartner komplexieren und dessen Aktivität hemmen kann. Die erste Bestimmung wird daher zweckmäßigerweise in Abwesenheit von Substanzen, wie etwa Heparin, durchgeführt, die eine Komplexbildung zwischen AT und AT-Bindepartner beschleunigen. Gegebenenfalls können Antagonisten für den Beschleuniger, beispielsweise Heparin-Antagonisten, wie etwa Polybren, in geringen Mengen zugesetzt werden. Die Zugabe von Heparin-Antagonisten ist insbesondere dann sinnvoll, wenn ein Patient zuvor mit Heparin behandelt worden ist, so dass eine vorhandene (geringe) Heparinkonzentration in der Probe zu einer ungewollten Beschleunigung der Komplexbildung zwischen AT und AT-Bindepartner führt. Die Zugabe von Antagonisten kann diese ungewollte Beschleunigung zumindest teilweise verhindern.

Nach der ersten Bestimmung des freien AT-Bindepartners wird der Reaktionsmischung vorzugsweise ein weiteres Reagenz zugesetzt, das einen Beschleuniger der Komplexbildung enthält, beispielsweise Heparin. Anschließend erfolgt die Bestimmung einer zweiten Aktivität unter Bedingungen, bei denen in der Probe vorhandenes AT den AT-Bindepartner komplexieren kann. Der AT-Gehalt in der Probe kann aus der Differenz der ersten und der zweiten Bestimmung ermittelt werden. Das gemessene Signal ist umgekehrt proportional zur AT-Konzentration in der Probe. Falls erforderlich, kann das dritte Reagenz weiterhin zusätzlichen AT-Bindepartner enthalten. Außerdem kann in einem weiteren Schritt zusätzliches Substrat pipettiert werden, sofern in der ersten Bestimmung bereits zuviel Substrat verbraucht worden ist.

Die Bestimmung des AT-Bindepartners erfolgt nach grundsätzlich bekannten Methoden, beispielsweise wie im Antithrombin III-Test von Roche Diagnostics GmbH, Mannheim, Deutschland, beschrieben. Die Bestimmung kann z.B. einen kinetischen Test oder eine Zweipunktbestimmung umfassen.

Weiterhin betrifft die Erfindung einen Reagenzienkit zum quantitativen Nachweis von AT in einer Probe, umfassend:
(a) ein erstes Reagenz R1 enthaltend einen AT-Bindepartner,
(b) ein zweites Reagenz R2 zu einer Bestimmung des freien AT-Bindepartners und
(c) ein drittes Reagenz R3 enthaltend einen Beschleuniger für die Interaktion zwischen AT und AT-Bindepartner, wobei das dritte Reagenz R3 separat vom ersten Reagenz R1 ist.

Das erste Reagenz R1 ist frei von einem Beschleuniger für die Interaktion, beispielsweise Komplexbildung, zwischen AT und AT-Bindepartner. Gegebenenfalls kann das erste Reagenz auch einen Antagonisten für einen derartigen Beschleuniger enthalten. Das zweite Reagenz R2 zur Bestimmung des AT-Bindepartner kann ein für die chromogene Bestimmung geeignetes Reagenz sein, das beispielsweise ein Substrat für den AT-Bindepartner enthält. Weiterhin kann das zweite Reagenz jedoch auch für die immunologische Bestimmung geeignet sein und beispielsweise Antikörper gegen einen freien ungebundenen AT-Bindepartner und gegebenenfalls weitere Reagenzien zur Durchführung eines immunologischen Tests, z.B. eines Latex-Tests, enthalten. Das dritte Reagenz R3 ist getrennt vom ersten Reagenz R1 und enthält einen Beschleuniger für die Interaktion, beispielsweise Komplexbildung zwischen AT und AT-Bindepartner, vorzugsweise Heparin.

Die Bestimmung kann an gängigen Analyseautomaten, wie etwa Hitachi, z.B. Hitachi-Modell 911, und Integra durchgeführt werden.

Weiterhin soll das erfindungsgemäße Verfahren durch das nachfolgende Beispiel erläutert werden:

### Bestimmung von Antithrombin III in Gegenwart von Lepirudin

### 1. Reaktionsschema

3 µl Probenlösung wurden in eine Messküvette pippetiert. Dazu wurden 175 µl Reagenz R1 pippetiert. Das Reagenz R1 besteht aus 100 mM Tris-HCI, 270 mM NaCI, 12 mM EDTA, 10 g/l Polyethylenglycol 6000, 1 g/l Rinderserumalbumin, 0,5 NIH/ml Rinderthrombin und einer geeigneten Menge eines Fibrinpolymerisations-Inhibitors, wie GPAP, pH 8,10. Anschließend erfolgte eine Inkubation für 5 min und danach die Zugabe von 75 µl Reagenz R2 (Chromozym TH 1,9 mM). Dann wurde durch kontinuierliche bichromatische Messung bei 415 und 700 nm (Primär- bzw. Sekundärwellenlänge) in einem kinetischen Test die erste Thrombinaktivität bestimmt.

Schließlich wurden 175µl Reagenz R3 (100 mM Tris-HCI, pH 8,1; Heparin 2 USP-E/ml; Rinderthrombin (3,5 NIH/ml; 140 mM NaCI) zugegeben und durch kontinierliche Messung in einem kinetischen Test die zweite Thrombinaktivität bestimmt. Der AT-Gehalt wurde aus der Differenz der zweiten und ersten Thrombinaktivität entsprechend der Vorschrift des Antithrombin III-Kits von Roche Diagnostics GmbH, Mannheim, ermittelt.

Diese Bestimmung wurde in Gegenwart unterschiedlicher Mengen an Hirudin (Lepirudin®, Fa. Behringwerke) (0, 1, 2, 4 und 8 µg/ml) durchgeführt.

Die Ergebnisse sind in der nachfolgenden Tabelle gezeigt.

| Lepirudin^{®} (µg/ml) | AT-konzentration (%) gefunden (Erfindung) | Abweichung (%) mit Test gemäß Erfindung | Abweichung (%) mit Test gemäß Stand der Technik |
|---|---|---|---|
| 0 | 100 | -- | -- |
| 1 | 100 | 0 | 5 |
| 2 | 99,9 | 0 | 10 |
| 4 | 102,5 | 3 | 25 |
| 8 | 130,0 | 30 | 52 |

Aus der Tabelle ist ersichtlich, dass die Störung durch Lepirudin® bis zu einer Konzentration von 4 µg/ml vollständig (Abweichung <5 %) beseitigt werden kann. Die therapeutischen Konzentrationen bei Verabreichung von Lepirudin® liegen üblicherweise innerhalb dieses Bereichs, so dass durch das erfindungsgemäße Verfahren eine zuverlässige Bestimmung des AT-Gehalts auch in Gegenwart von potentiell störenden Arzneimitteln erreicht wird.

## Patentansprüche

1. Verfahren zum Nachweis von Antithrombin III (AT) in einer Probe, die eventuell einen Störfaktor enthält, umfassend die Schritte:
(a) Inkontaktbringen der Probe mit einem ersten Reagenz R1 enthaltend einen AT-Bindepartner unter Bedingungen, bei denen der AT-Bindepartner im Wesentlichen nicht mit AT interagiert, aber mit dem Störfaktor interagiert,
(b) Zugeben eines zweiten Reagenz R2 zu einer ersten Bestimmung des freien Anteils des AT-Bindepartners,
(c) Zugeben eines dritten Reagenz R3 zur Änderung der Bedingungen, so dass der AT-Bindepartner mit AT interagiert, und zweite Bestimmung des freien Anteils des AT-Bindepartners und
(d) Ermitteln des AT-Gehalts in der Probe aus der Differenz der ersten und zweiten Bestimmung des freien Anteils des AT-Bindepartners.

2. Verfahren nach Anspruch 1,
**dadurch gekennzeichnet,**
**dass** das erste Reagenz R1 einen AT-Bindepartner ausgewählt aus Thrombin und Faktor Xa enthält.

3. Verfahren nach Anspruch 2,
**dadurch gekennzeichnet,**
**dass** das erste Reagenz R1 als AT-Bindepartner Thrombin enthält.

4. Verfahren nach einem der Ansprüche 1 bis 3,
**dadurch gekennzeichnet,**
**dass** das zweite Reagenz R2 ein chromogenes Substrat zur Bestimmung von freiem AT-Bindepartner enthält.

5. Verfahren nach einem der Ansprüche 1 bis 3,
**dadurch gekennzeichnet,**
**dass** das zweite Reagenz R2 einen Antikörper zur Bestimmung von freiem AT-Bindepartner enthält.

6. Verfahren nach einem der Ansprüche 1 bis 5,
**dadurch gekennzeichnet,**
**dass** das dritte Reagenz R3 einen Beschleuniger der Interaktion zwischen AT und AT-Bindepartner enthält.

7. Verfahren nach Anspruch 6,
**dadurch gekennzeichnet,**
**dass** das dritte Reagenz R3 Heparin als Beschleuniger enthält.

8. Verfahren nach einem der Ansprüche 1 bis 7,
**dadurch gekennzeichnet,**
**dass** das erste Reagenz R1 weiterhin einen Antagonisten für einen Beschleuniger der Interaktion zwischen AT und AT-Bindepartner enthält.

9. Verfahren nach Anspruch 8,
**dadurch gekennzeichnet,**
**dass** das erste Reagenz R1 Polybren als Antagonisten von Heparin enthält.

10. Verfahren nach einem der Ansprüche 1 bis 9,
**dadurch gekennzeichnet,**
**dass** das dritte Reagenz R3 weiterhin zusätzlichen AT-Bindepartner enthält.

11. Verfahren nach einem der Ansprüche 1 bis 10,
**dadurch gekennzeichnet,**
**dass** die Bestimmung des AT-Bindepartners eine kinetische Bestimmung umfasst.

12. Verfahren zum Nachweis von Antithrombin III (AT) in einer Probe, basierend auf der Bestimmung der Interaktion eines AT-Bindepartners mit in der Probe vorhandenem AT,
**dadurch gekennzeichnet,**
**dass** man eine erste Bestimmung des AT-Bindepartners ohne Interaktion mit AT und danach eine zweite Bestimmung des AT-Bindepartners mit AT-Interaktion durchführt und den AT-Gehalt in der Probe aus der Differenz der ersten und zweiten Bestimmung ermittelt.

13. Verwendung eines Reagenzienkits in einem Verfahren nach einem der Ansprüche 1-12, wobei der Reagenzienkit umfasst:
(a) ein erstes Reagenz R1 enthaltend einen AT-Bindepartner,
(b) ein zweites Reagenz R2 zu einer Bestimmung des freien AT-Bindepartners und
(c) ein drittes Reagenz R3 enthaltend einen Beschleuniger für die Interaktion zwischen AT und AT-Bindepartner, wobei das dritte Reagenz R3 separat vom ersten Reagenz R1 ist.

14. Verwendung nach Anspruch 13,
wobei das zweite Reagenz R2 für eine chromogene Bestimmung des AT-Bindepartners geeignet ist.

15. Verwendung nach Anspruch 13,
wobei das zweite Reagenz R2 für eine immunologische Bestimmung des AT-Bindepartners geeignet ist.

## Claims

1. Method for detecting antithrombin III (AT) in a sample which may contain an interfering factor comprising the steps:
(a) contacting the sample with a first reagent R1 containing an AT binding partner under conditions where the AT binding partner essentially does not interact with AT but interacts with the interfering factor,
(b) adding a second reagent R2 for a first determination of the free fraction of the AT binding partner,
(c) adding a third reagent R3 to change the conditions such that the AT binding partner interacts with AT and carrying out a second determination of the free fraction of AT binding partner and
(d) determining the AT content in the sample from the difference between the first and second determination of the free fraction of the AT binding partner.

2. Method as claimed in claim 1,
**characterized in that**
the first reagent R1 contains an AT binding partner selected from thrombin and factor Xa.

3. Method as claimed in claim 2,
**characterized in that**
the first reagent R1 contains thrombin as the AT binding partner.

4. Method as claimed in one of the claims 1 to 3,
**characterized in that**
the second reagent R2 contains a chromogenic substrate for the determination of the free AT binding partner.

5. Method as claimed in one of the claims 1 to 3,
**characterized in that**
the second reagent R2 contains an antibody for determining the free AT binding partner.

6. Method as claimed in one of the claims 1 to 5,
**characterized in that**
the third reagent R3 contains an accelerator of the interaction between AT and AT binding partner.

7. Method as claimed in claim 6,
**characterized in that**
the third reagent R3 contains heparin as the accelerator.

8. Method as claimed in one of the claims 1 to 7,
**characterized in that**
the first reagent R1 additionally contains an antagonist for an accelerator of the interaction between AT and AT binding partner.

9. Method as claimed in claim 8,
**characterized in that**
the first reagent R1 contains polybrene as a heparin antagonist.

10. Method as claimed in one of the claims 1 to 9,
**characterized in that**
the third reagent R3 also contains an additional AT binding partner.

11. Method as claimed in one of the claims 1 to 10,
**characterized in that**
the determination of the AT binding partner comprises a kinetic determination.

12. Method for the detection of antithrombin III (AT) in a sample based on determining the interaction of an AT binding partner with AT present in the sample,
**characterized in that**
a first determination of the AT binding partner is carried out without interaction with AT and subsequently a second determination of the AT binding partner is carried out with AT interaction and the AT content of the sample is determined from the difference between the first and second determination.

13. Use of a reagent kit in a method as claimed in one of the claims 1-12,
wherein the reagent kit comprises:
(a) a first reagent R1 containing an AT binding partner,
(b) a second reagent R2 to determine the free AT binding partner and
(c) a third reagent R3 containing an accelerator for the interaction between AT and AT binding partner where the third reagent R3 is separate from the first reagent R1.

14. Use as claimed in claim 13,
wherein the second reagent R2 is suitable for a chromogenic determination of the AT binding partner.

15. Use as claimed in claim 13,
wherein the second reagent R2 is suitable for an immunological determination of the AT binding partner.

## Revendications

1. Procédé pour la détermination de l'antithrombine III (AT) dans un échantillon, qui contient éventuellement un facteur parasite, comprenant les étapes consistant à :
(a) mettre en contact l'échantillon avec un premier réactif R1 contenant un partenaire de liaison AT, dans des conditions dans lesquelles le partenaire de liaison AT n'interagit sensiblement pas avec AT mais interagit avec le facteur parasite,
(b) ajouter un deuxième réactif R2 pour une première détermination de la partie libre du partenaire de liaison AT,
(c) ajouter un troisième réactif R3 pour modifier les conditions, de sorte que le partenaire de liaison AT interagisse avec AT, et une deuxième détermination de la partie libre du partenaire de liaison AT et
(d) déterminer la teneur en AT dans l'échantillon à partir de la différence entre la première et la seconde détermination de la partie libre du partenaire de liaison AT.

2. Procédé selon la revendication 1,
**caractérisé en ce que**
le premier réactif R1 contient un partenaire de liaison AT choisi parmi la thrombine et le facteur Xa.

3. Procédé selon la revendication 2,
**caractérisé en ce que**
le premier réactif R1 contient de la thrombine en tant que partenaire de liaison AT.

4. Procédé selon l'une des revendications 1 à 3,
**caractérisé en ce que**
le deuxième réactif R2 contient un substrat chromogène pour la détermination de partenaire de liaison AT libre.

5. Procédé selon l'une des revendications 1 à 3,
**caractérisé en ce que**
le deuxième réactif R2 contient un anticorps pour la détermination de partenaire de liaison AT libre.

6. Procédé selon l'une des revendications 1 à 5,
**caractérisé en ce que**
le troisième réactif R3 contient un accélérateur de l'interaction entre AT et le partenaire de liaison AT.

7. Procédé selon la revendication 6,
**caractérisé en ce que**
le troisième réactif R3 contient de l'héparine en tant qu'accélérateur.

8. Procédé selon l'une des revendications 1 à 7,
**caractérisé en ce que**
le premier réactif R1 contient de plus un antagoniste pour un accélérateur de l'interaction entre AT et le partenaire de liaison AT.

9. Procédé selon la revendication 8,
**caractérisé en ce que**
le premier réactif R1 contient du polybrène en tant qu'antagoniste de l'héparine.

10. Procédé selon l'une des revendications 1 à 9,
**caractérisé en ce que**
le troisième réactif R3 contient de plus du partenaire de liaison AT supplémentaire.

11. Procédé selon l'une des revendications 1 à 10,
**caractérisé en ce que**
la détermination du partenaire de liaison AT comprend une détermination cinétique.

12. Procédé pour déterminer l'antithrombine III (AT) dans un échantillon, basé sur la détermination de l'interaction d'un partenaire de liaison AT avec l'AT présent dans l'échantillon,
**caractérisé en ce que**
l'on procède à une première détermination du partenaire de liaison AT sans interaction avec AT et ensuite une deuxième détermination du partenaire de liaison AT avec l'interaction AT, et on détermine la teneur en AT dans l'échantillon à partir de la différence entre la première et la seconde détermination.

13. Utilisation d'un kit de réactif dans un procédé selon l'une des revendications 1-12, le kit de réactif comprenant :
(a) un premier réactif R1 contenant un partenaire de liaison AT,
(b) un second réactif R2 pour la détermination du partenaire de liaison AT libre et
(c) un troisième réactif R3 contenant un accélérateur pour l'interaction entre AT et le partenaire de liaison AT, le troisième réactif R3 étant séparé du premier réactif R1.

14. Utilisation selon la revendication 13,
dans laquelle le second réactif R2 est approprié à une détermination chromogène du partenaire de liaison AT.

15. Utilisation selon la revendication 13,
dans laquelle le second réactif R2 est approprié à une détermination immunologique du partenaire de liaison AT.
